# EUROPEAN PATENT APPLICATION

(11) **EP 4 840 850 A1**
(43) Date of publication of application: **18.11.2026**
(21) Application number: 25744621.1
(22) Date of filing: 22.01.2025
(51) Int. Cl.: C07D 221/14, C09K 11/06, C09K 9/02

(54) **NAPHTHALIMIDE-BASED SCHIFF BASE DERIVATIVE, AND PREPARATION METHOD THEREFOR AND USE THEREOF**

(30) Priority: 25.01.2024 CN 202410110064
(71) Applicant: Anhui Easpeed Technology Co., Ltd., Hefei, Anhui 230088 (CN)
(72) Inventor: ZHANG, Liangliang, Hefei, Anhui 230088 (CN); WANG, Haiqian, Hefei, Anhui 230088 (CN); WANG, Hui, Hefei, Anhui 230088 (CN); ZHANG, Xudong, Hefei, Anhui 230088 (CN); YU, Zengchao, Hefei, Anhui 230088 (CN); HAN, Dongcheng, Hefei, Anhui 230088 (CN)
(74) Representative: Wilson Gunn
(86) International application number: PCT/CN2025/073994
(87) International publication number: WO 2025/157183

(57) **Abstract**

Disclosed are a naphthalimide-based Schiff base derivative, and a preparation method and a use thereof. The naphthalimide-based Schiff base derivative has a structural formula of formula (1) or formula (2), where R₁, R₂, R₃, and R₄ are each independently H or alkyl having 1 to 4 carbon atoms.

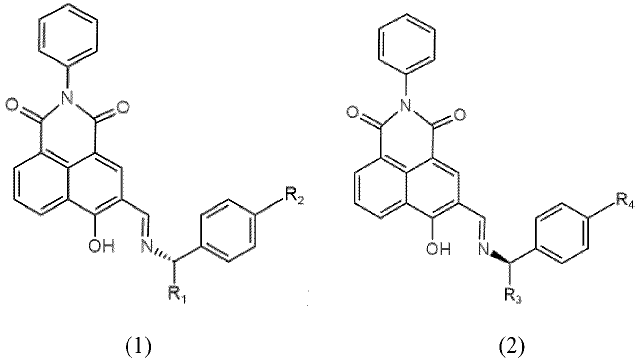

## Description

### FIELD

The present disclosure relates to the technical field of organic luminescent materials, and more particularly, to a naphthalimide-based Schiff base derivative, and a preparation method and use thereof.

### BACKGROUND

Static volumetric three-dimensional (3D) display is a very important branch of three-dimensional display technologies. In general, volumetric pixel (voxel) luminescence is generated by addressing light beams within a display medium, and volumetric three-dimensional patterns are formed through arrays of multiple voxel points.

Among currently reported methods, static volumetric three-dimensional display is commonly achieved by using upconversion luminescence of rare earth materials or laser ionization of air. However, these technologies require expensive high-energy light beams and involve significant safety risks. Photochromism refers to a phenomenon in which a material A undergoes a specific transformation under the action of light to produce an isomer B with different properties, and can revert under the action of another light beam or heat. Since high-energy light beams are not required, photochromic materials are also capable of achieving light-addressed voxel luminescence when the reaction rate is sufficiently fast. Among such materials, salicylaldehyde primary amine Schiff base molecules undergo rapid intramolecular proton transfer upon excitation, thereby exhibiting photochromic and thermochromic phenomena, and thus can be applied in fields such as information storage, optical switches, and information display. However, the keto structure formed after photochromic transformation of salicylaldehyde primary amine Schiff bases exhibits relatively low luminescence intensity, thereby hindering their applications in static volumetric three-dimensional display systems.

### SUMMARY

The present disclosure aims to solve at least one of the technical problems in the related art to a certain extent. To this end, an objective of the present disclosure is to provide a naphthalimide-based Schiff base derivative, and a preparation method and use thereof. The naphthalimide-based Schiff base derivative can enhance the luminescence intensity of the keto isomer of the Schiff base, and thus can serve as a display medium for static volumetric three-dimensional display and can improve the luminescence intensity of voxel points generated through light-beam addressing.

In a first aspect of the present disclosure, the present disclosure provides a naphthalimide-based Schiff base derivative. According to an embodiment of the present disclosure, the naphthalimide-based Schiff base derivative has a structural formula of:

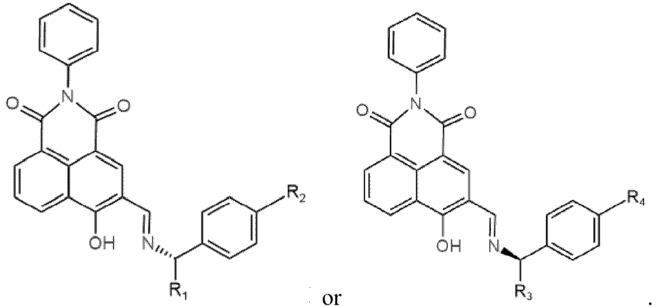

R₁, R₂, R₃, and R₄ are each independently H or alkyl having 1 to 4 carbon atoms.

According to the above-described embodiments of the present disclosure, the naphthalimide-based Schiff base derivative has an enol structure and possesses photochromic properties. Based on the principle of excited-state proton transfer, the derivative can rapidly isomerize from the enol form into the keto form under ultraviolet light stimulation, accompanied by changes in the absorption spectrum and excitation wavelength. In addition, the keto isomer is a metastable structure that can quickly revert to the enol form. When ultraviolet light intersects with excitation light for the keto isomer, voxel luminescence can be generated in a three-dimensional space. When the ultraviolet light or the excitation light for the keto isomer is turned off, the voxel point immediately disappears, thereby enabling dual-beam addressing within the medium space. Moreover, the 1,8-naphthalimide group in the aldehyde structure exhibits strong fluorescence, and the phenyl group connected to the nitrogen atom in the 1,8-naphthalimide structure is a rigid group capable of reducing non-radiative electron transitions. Accordingly, the luminescence intensity of the keto isomer of the Schiff base can be enhanced, such that the derivative can serve as a display medium for static volumetric three-dimensional display, thereby improving the luminescence intensity of voxel points generated through light-beam addressing. Furthermore, the generated voxel luminescence exhibits high contrast, high refresh speed, and good stability.

In a second aspect of the present disclosure, the present disclosure provides a method for preparing the above-described naphthalimide-based Schiff base derivative. The method includes: performing a first reaction on a first compound with N-(3-formyl-4-hydroxy-1,8-naphthodicarboxyl)aniline in a first organic solvent to obtain the naphthalimide-based Schiff base derivative. The first compound has a structural formula of:

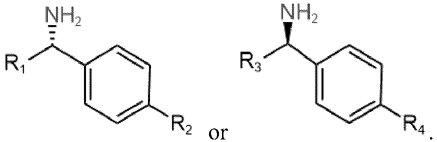

R₁, R₂, R₃, and R₄ are each independently H or alkyl having 1 to 4 carbon atoms.

Accordingly, the naphthalimide-based Schiff base derivative prepared by the method can enhance the luminescence intensity of the keto isomer of the Schiff base, such that the derivative can serve as a display medium for static volumetric three-dimensional display, thereby improving the luminescence intensity of voxel points generated through light-beam addressing. Furthermore, the generated voxel luminescence exhibits high contrast, high refresh speed, and good stability. Moreover, the method provides high yield and low cost, and is suitable for industrial production.

In addition, according to the above-described embodiments of the present disclosure, the method for preparing the above-described naphthalimide-based Schiff base derivative may also have the following additional technical features:

In some embodiments of the present disclosure, a molar ratio of N-(3-formyl-4-hydroxy-1,8-naphthodicarboxyl)aniline to the first compound is 1:(0.9 to 1.1).

In some embodiments of the present disclosure, the first organic solvent includes at least one of methanol, ethanol, or dimethyl sulfoxide.

In some embodiments of the present disclosure, N-(3-formyl-4-hydroxy-1,8-naphthodicarboxyl)aniline is prepared by the following steps: (1) performing a second reaction on 4-bromo-1,8-naphthalic anhydride and aniline in a first acidic solvent to obtain a second compound; (2) performing a third reaction on the second compound and a methoxy-containing reagent in a second organic solvent containing a catalyst to obtain a third compound; (3) performing a fourth reaction on the third compound and hydrohalic acid to obtain a fourth compound; and (4) performing a fifth reaction on the fourth compound and hexamethylenetetramine in a second acidic solvent to obtain N-(3-formyl-4-hydroxy-1,8-naphthodicarboxyl)aniline.

In some embodiments of the present disclosure, the first acidic solvent and the second acidic solvent each independently include glacial acetic acid and/or trifluoroacetic acid.

In some embodiments of the present disclosure, in step (1), a molar ratio of 4-bromo-1,8-naphthalic anhydride to aniline is 1:(1.5 to 3.5).

In some embodiments of the present disclosure, in step (2), a molar ratio of the second compound, the methoxy-containing reagent, and the catalyst is 1:(7 to 8):(0.1 to 0.3).

In some embodiments of the present disclosure, the second organic solvent includes at least one of methanol, N,N-dimethylformamide, or dimethyl sulfoxide.

In some embodiments of the present disclosure, the methoxy-containing reagent includes sodium methoxide and/or potassium methoxide.

In some embodiments of the present disclosure, the catalyst includes at least one of copper sulfate, copper phosphate, copper acetate, or copper nitrate.

In some embodiments of the present disclosure, in step (3), the hydrohalic acid includes HI and/or HBr.

In some embodiments of the present disclosure, a concentration of the hydrohalic acid is not less than 57 wt%.

In some embodiments of the present disclosure, a content of the third compound in the hydrohalic acid ranges from 60 mmol/L to 100 mmol/L.

In some embodiments of the present disclosure, in step (4), a molar ratio of the fourth compound to hexamethylenetetramine is 1:(1 to 3).

In a third aspect of the present disclosure, the present disclosure provides use of the above-described naphthalimide-based Schiff base derivative in a static volumetric three-dimensional display system. Accordingly, the problem regarding the luminescence intensity of the keto isomer of Schiff bases in applications of static volumetric 3D display systems can be solved.

Additional aspects and advantages of the present disclosure will be provided in part in the following description, or will become apparent in part from the following description, or can be learned from practicing of the present disclosure.

### BRIEF DESCRIPTION OF THE DRAWINGS

The above and/or additional aspects and advantages of the present disclosure will become apparent and readily understood from the following description of embodiments taken in conjunction with the accompanying drawings.
FIG. 1 is a schematic flowchart of a method for preparing N-(3-formyl-4-hydroxy-1,8-naphthodicarboxyl)aniline according to an embodiment of the present disclosure.
FIG. 2 is a flowchart of a method for preparing a naphthalimide-based Schiff base derivative according to Example 1 of the present disclosure.
FIG. 3 is a proton nuclear magnetic resonance spectrum of the target product obtained in Example 1 of the present disclosure.
FIG. 4 is an absorption spectrum of the target product obtained in Example 1 of the present disclosure.
FIG. 5 shows voxel luminescence inside a solution prepared in Example 3 of the present disclosure.

### DETAILED DESCRIPTION

Embodiments of the present disclosure will be described in detail below. The embodiments described below are illustrative only, and are intended to explain, rather than limit, the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

In a first aspect of the present disclosure, the present disclosure provides a naphthalimide-based Schiff base derivative. According to an embodiment of the present disclosure, the naphthalimide-based Schiff base derivative has a structural formula of:

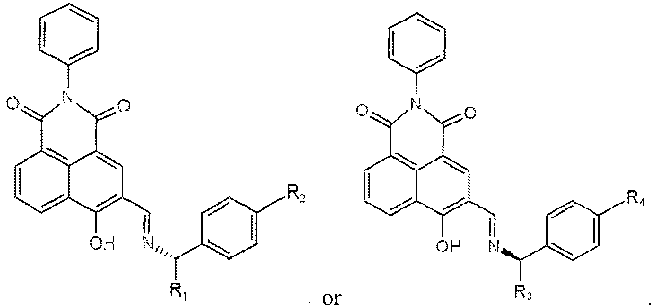

R₁, R₂, R₃, and R₄ are each independently H or alkyl having 1 to 4 carbon atoms.

According to the above-described embodiments of the present disclosure, the naphthalimide-based Schiff base derivative has an enol structure and possesses photochromic properties. Based on the principle of excited-state proton transfer, the derivative can rapidly isomerize from the enol form into the keto form under ultraviolet light stimulation, accompanied by changes in the absorption spectrum and excitation wavelength. In addition, the keto isomer is a metastable structure that can quickly revert to the enol form. When ultraviolet light intersects with excitation light for the keto isomer, voxel luminescence can be generated in a three-dimensional space. When the ultraviolet light or the excitation light for the keto isomer is turned off, the voxel point immediately disappears, thereby enabling dual-beam addressing within the medium space. Moreover, the 1,8-naphthalimide group in the aldehyde structure exhibits strong fluorescence, and the phenyl group connected to the nitrogen atom in the 1,8-naphthalimide structure is a rigid group capable of reducing non-radiative electron transitions. Accordingly, the luminescence intensity of the keto isomer of the Schiff base can be enhanced, such that the derivative can serve as a display medium for static volumetric three-dimensional display, thereby improving the luminescence intensity of voxel points generated through light-beam addressing. Furthermore, the generated voxel luminescence exhibits high contrast, high refresh speed, and good stability.

In a second aspect of the present disclosure, the present disclosure provides a method for preparing the above-described naphthalimide-based Schiff base derivative. The method includes: performing a first reaction on a first compound and N-(3-formyl-4-hydroxy-1,8-naphthodicarboxyl)aniline in a first organic solvent to obtain the naphthalimide-based Schiff base derivative. N-(3-formyl-4-hydroxy-1,8-naphthodicarboxyl)aniline has a structural formula of: 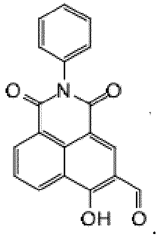 The first compound has a structural formula of: 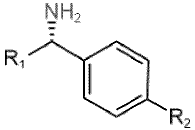 or 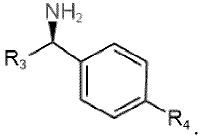 R₁, R₂, R₃, and R₄ are each independently H or alkyl having 1 to 4 carbon atoms. Accordingly, the naphthalimide-based Schiff base derivative prepared by the method can enhance the luminescence intensity of the keto isomer of the Schiff base, such that the derivative can serve as a display medium for static volumetric three-dimensional display, thereby improving the luminescence intensity of voxel points generated through light-beam addressing. Furthermore, the generated voxel luminescence exhibits high contrast, high refresh speed, and good stability. Moreover, the method provides high yield and low cost, and is suitable for industrial production.

Specifically, the first compound and N-(3-formyl-4-hydroxy-1,8-naphthodicarboxyl)aniline may be mixed in the first organic solvent and refluxed for 2 hours to 6 hours to obtain the naphthalimide-based Schiff base derivative. Accordingly, the progress of the first reaction is facilitated, thereby improving the utilization rate of the reaction raw materials.

According to specific embodiments of the present disclosure, the molar ratio of N-(3-formyl-4-hydroxy-1,8-naphthodicarboxyl)aniline to the first compound may be 1:(0.9 to 1.1), for example, 1:0.9, 1:0.92, 1:0.94, 1:0.96, 1:0.98, 1:1, 1:1.02, 1:1.04, 1:1.06, 1:1.08, or 1:1.1. The inventors have discovered that an excessively high or low molar ratio of N-(3-formyl-4-hydroxy-1,8-naphthodicarboxyl)aniline to the first compound may reduce the yield of the first reaction. In the present disclosure, by controlling the molar ratio of N-(3-formyl-4-hydroxy-1,8-naphthodicarboxyl)aniline to the first compound within the above-described range, the yield of the first reaction can be increased.

It should be noted that the specific composition of the first organic solvent is not particularly limited, and may be selected by those skilled in the art according to actual requirements. For example, the first organic solvent may include at least one of methanol, ethanol, or dimethyl sulfoxide.

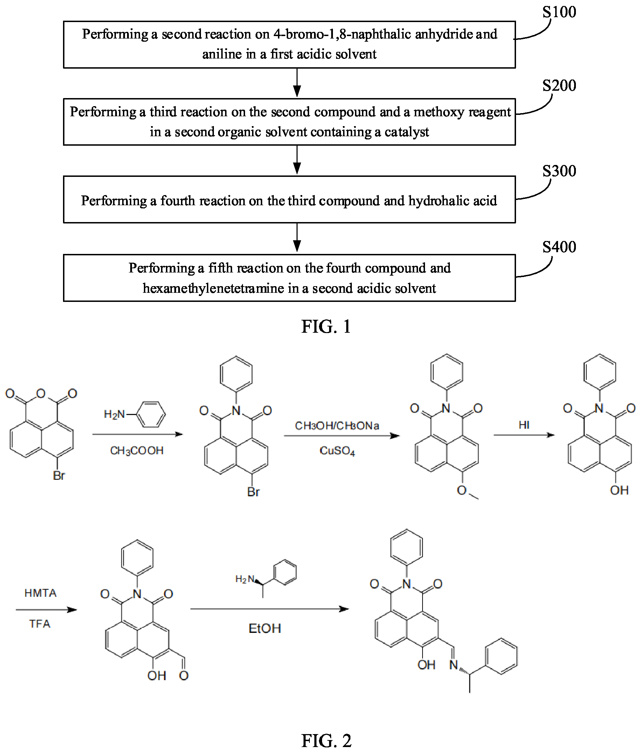

According to specific embodiments of the present disclosure, with reference to FIG. 1, N-(3-formyl-4-hydroxy-1,8-naphthodicarboxyl)aniline may be prepared by the following steps.

S100: Performing a second reaction on 4-bromo-1,8-naphthalic anhydride and aniline in a first acidic solvent

In this step, 4-bromo-1,8-naphthalic anhydride and aniline are subjected to a second reaction in a first acidic solvent to obtain a second compound. The second compound has a structural formula of:

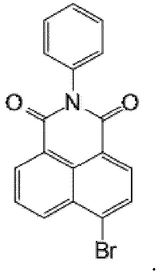

Specifically, 4-bromo-1,8-naphthalic anhydride and aniline may be mixed in the first acidic solvent and refluxed for 4 hours to 8 hours under protection of an inert gas (such as nitrogen) to obtain the second compound. Accordingly, the progress of the second reaction is facilitated, thereby improving the utilization rate of the reaction raw materials.

According to specific embodiments of the present disclosure, the molar ratio of 4-bromo-1,8-naphthalic anhydride to aniline may be 1:(1.5 to 3.5), for example, 1:1.5, 1:1.7, 1:2, 1:2.3, 1:2.5, 1:2.7, 1:3, 1:3.3, or 1:3.5. In the present disclosure, by controlling the molar ratio of 4-bromo-1,8-naphthalic anhydride to aniline within the above-described range, the yield of the second reaction can be increased.

It should be noted that the specific composition of the first acidic solvent is not particularly limited, and can be selected by those skilled in the art according to actual requirements. For example, the first acidic solvent may include glacial acetic acid and/or trifluoroacetic acid (TFA).

S200: Performing a third reaction on the second compound and a methoxy-containing reagent in a second organic solvent containing a catalyst

In this step, the second compound and a methoxy-containing reagent are subjected to a third reaction in a second organic solvent containing the catalyst to obtain a third compound. The third compound has a structural formula of:

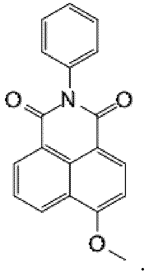

Specifically, the second compound and the methoxy-containing reagent may be mixed in the second organic solvent and refluxed for 6 hours to 10 hours under protection of an inert gas (such as nitrogen) to obtain the third compound. Accordingly, the progress of the third reaction is facilitated, thereby improving the utilization rate of the reaction raw materials.

According to specific embodiments of the present disclosure, the molar ratio of the second compound, the methoxy-containing reagent, and the catalyst may be 1:(7 to 8):(0.1 to 0.3), for example, 1:7:0.1, 1:7:0.2, 1:7:0.3, 1:8:0.1, 1:8:0.2, or 1:8:0.3. In the present disclosure, by controlling the molar ratio of the second compound, the methoxy-containing reagent, and the catalyst within the above-described range, the yield of the third reaction can be increased.

Specifically, the methoxy-containing reagent may include sodium methoxide and/or potassium methoxide. The second organic solvent may include at least one of methanol, N,N-dimethylformamide, or dimethyl sulfoxide. The catalyst may include at least one of copper sulfate, copper phosphate, copper acetate, or copper nitrate.

S300: Performing a fourth reaction on the third compound and hydrohalic acid

In this step, the third compound and hydrohalic acid are subjected to a fourth reaction to obtain a fourth compound. The hydrohalic acid may include HI and/or HBr. The fourth compound has a structural formula of:

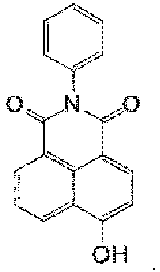

Specifically, the third compound and the hydrohalic acid may be mixed and refluxed for 8 hours to 12 hours under protection of an inert gas (such as nitrogen) to obtain the fourth compound. Accordingly, the progress of the fourth reaction is facilitated, thereby improving the utilization rate of the reaction raw materials.

According to specific embodiments of the present disclosure, the concentration of the hydrohalic acid is not less than 57 wt%. Accordingly, the progress of the fourth reaction is promoted, thereby improving the utilization rate of the reaction raw materials.

According to specific embodiments of the present disclosure, the content of the third compound in the hydrohalic acid may range from 60 mmol/L to 100 mmol/L, for example, 60 mmol/L, 65 mmol/L, 70 mmol/L, 75 mmol/L, 80 mmol/L, 85 mmol/L, 90 mmol/L, 95 mmol/L, or 100 mmol/L. In the present disclosure, by controlling the content of the third compound in the hydrohalic acid within the above-described range, the yield of the fourth reaction can be increased.

S400: Performing a fifth reaction on the fourth compound and hexamethylenetetramin in a second acidic solvent

In this step, the fourth compound and hexamethylenetetramine (HMTA) are subjected to a fifth reaction in a second acidic solvent to obtain N-(3-formyl-4-hydroxy-1,8-naphthodicarboxyl)aniline. The second acidic solvent may include glacial acetic acid and/or trifluoroacetic acid.

Specifically, a mixture of the fourth compound and hexamethylenetetramine may be refluxed in glacial acetic acid and/or trifluoroacetic acid for 8 hours to 12 hours. Accordingly, the progress of the fifth reaction is facilitated, thereby improving the utilization rate of the reaction raw materials.

According to specific embodiments of the present disclosure, the molar ratio of the fourth compound to hexamethylenetetramine may be 1:(1 to 3), for example, 1:1, 1:1.2, 1:1.4, 1:1.6, 1:1.8, 1:2, 1:2.2, 1:2.4, 1:2.6, 1:2.8, or 1:3. In the present disclosure, by controlling the molar ratio of the fourth compound to hexamethylenetetramine within the above-described range, the yield of the fifth reaction can be increased.

In a third aspect of the present disclosure, the present disclosure provides use of the above-described naphthalimide-based Schiff base derivative in a static volumetric three-dimensional display system. Accordingly, the problem regarding the luminescence intensity of the keto isomer of Schiff bases in applications of static volumetric 3D display systems can be solved.

Specifically, the above-described naphthalimide-based Schiff base derivative may be dispersed in an organic solvent or cured in polymer materials such as epoxy resin, polymethyl methacrylate, or polydimethylsiloxane to serve as a volumetric three-dimensional display medium. The voxel excitation light source adopts two light sources with different wavelengths. The two light sources intersect within the display medium for addressing. When the scanning speed of the light sources exceeds the refresh rate of the human eye, arrays of multiple voxel points can be formed, thereby displaying a three-dimensional stereoscopic image.

The present disclosure is described below with reference to specific examples. It should be noted that these examples are merely illustrative and do not limit the present disclosure in any way.

### Example 1

As illustrated in FIG. 2, the method for preparing the naphthalimide-based Schiff base derivative is as follows:
27.7 g (0.1 mol) of 4-bromo-1,8-naphthalic anhydride and 18.6 g (0.2 mol) of aniline were mixed in 300 mL of glacial acetic acid and refluxed at 120°C for 6.5 hours under nitrogen protection. After cooling, the mixture was poured into water, producing a yellow precipitate. After filtration and recrystallization, pale yellow crystals were obtained as the second compound.

17.6 g (0.05 mol) of the second compound, 20.2 g (0.375 mol) of sodium methoxide, and 1.6 g (0.01 mol) of copper sulfate were mixed and dispersed in 200 mL of a dry methanol solution. The mixture was refluxed at 65°C for 8 hours under nitrogen protection. The resulting solution was cooled to room temperature and filtered to obtain a pale yellow solid. Then, the solid was washed with 10 wt% hydrochloric acid (30 mL*3) and water (20 mL*3) to obtain the third compound.

6.0 g (0.02 mol) of the third compound and 200 mL of 57 wt% hydroiodic acid were mixed and refluxed for 12 hours under nitrogen protection. The resulting solution was cooled to room temperature and filtered. The crude product was then washed with water (50 mL*3) to obtain a yellow-green solid. The crude product was purified by column chromatography to obtain the fourth compound.

A mixture of 2.9 g (0.01 mol) of the fourth compound and 2.8 g (0.02 mol) of hexamethylenetetramine was refluxed in trifluoroacetic acid for 8 hours, and the resulting reaction mixture was then cooled to room temperature. The reaction mixture was diluted with distilled water, and the formed precipitate was filtered. The precipitate was washed several times with distilled water and dried. Then, the dried precipitate was dissolved in dichloromethane and purified using a short silica plug to obtain N-(3-formyl-4-hydroxy-1,8-naphthodicarboxyl)aniline.

0.63 g (2 mmol) of N-(3-formyl-4-hydroxy-1,8-naphthodicarboxyl)aniline and 0.24 g (2 mmol) of (S)-α-methylbenzylamine were mixed in 20 mL of ethanol, refluxed for 4 hours, filtered, and washed to obtain the target compound.

### Example 2

The method for preparing the naphthalimide-based Schiff base derivative is as follows:
0.63 g (2 mmol) of N-(3-formyl-4-hydroxy-1,8-naphthodicarboxyl)aniline and 0.24 g (2 mmol) of (R)-α-methylbenzylamine were mixed in 20 mL of ethanol, refluxed for 4 hours, filtered, and washed to obtain the target compound.

The target products obtained in Examples 1 and 2 were subjected to proton nuclear magnetic resonance spectroscopy and ultraviolet-visible absorption spectroscopy analyses.

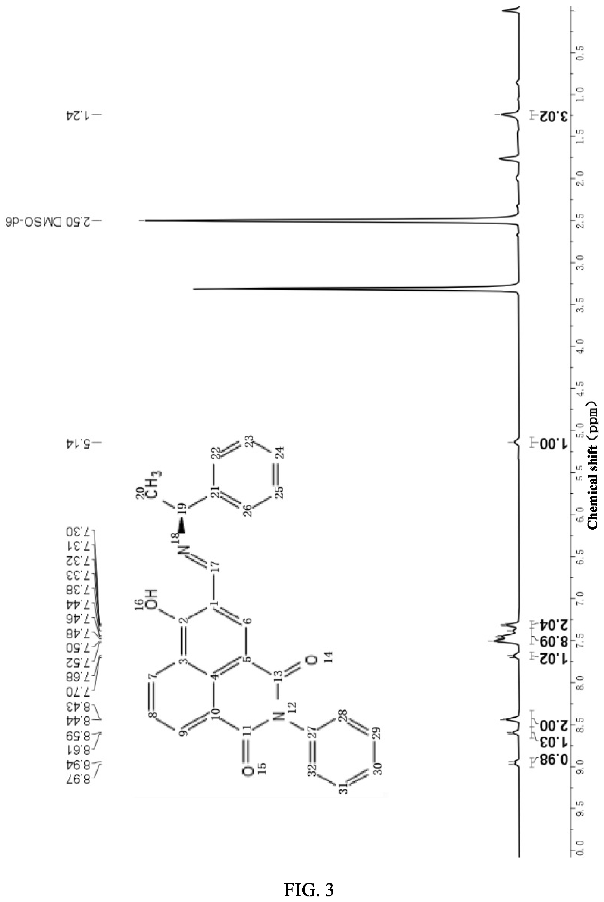

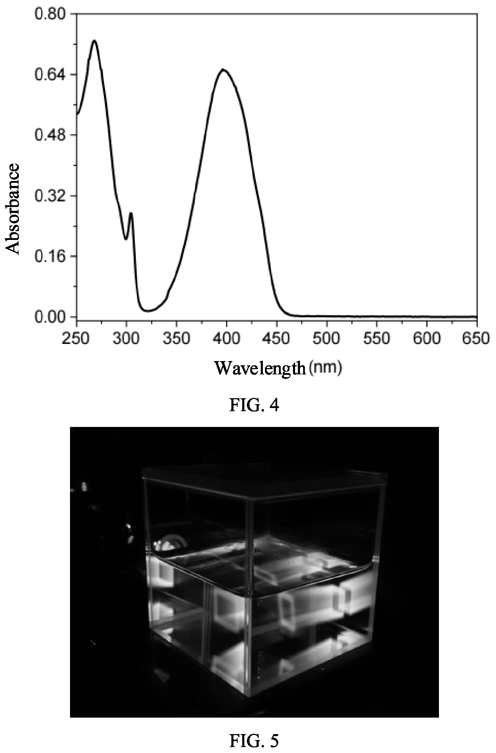

The proton nuclear magnetic resonance spectrum of the target product obtained in Example 1 is shown in FIG. 3. ¹H NMR (400 MHz, DMSO-d6), chemical shift (δ): 8.96 (d, J=12.7 Hz, 1H), 8.60 (d, J=6.8 Hz, 1H), 8.43 (d, J=6.8 Hz, 2H), 7.69 (d, J=9.7 Hz, 1H), 7.51 (d, J=7.2 Hz, 3H), 7.48-7.43 (m, 5H), 7.38 (s, 1H), 7.31 (t, J=5.8 Hz, 2H), 5.13 (d, J=9.6 Hz, 1H), 1.24 (s, 4H). The number and positions of the H atoms in the proton nuclear magnetic resonance spectrum correspond one-to-one with the number and positions of the H atoms in the naphthalimide-based Schiff base derivative, indicating that the naphthalimide-based Schiff base derivative prepared in Example 1 had the desired structure. The proton nuclear magnetic resonance spectrum of the target product obtained in Example 2 was similar to that of Example 1, indicating that the naphthalimide-based Schiff base derivative prepared in Example 2 also had the desired structure. The ultraviolet-visible absorption spectrum test results of the target product obtained in Example 1 are shown in FIG. 4. The ultraviolet-visible absorption spectrum test results of the target product obtained in Example 2 was similar to those of Example 1.

### Example 3

The volumetric three-dimensional application method of the naphthalimide-based Schiff base derivative is as follows:
The naphthalimide-based Schiff base derivative obtained in Example 1 was fully dissolved in cyclohexane to prepare a cyclohexane solution of the naphthalimide-based Schiff base derivative having a concentration of 0.01 mmol/L, and the solution was placed in a quartz vessel. As illustrated in FIG. 5, when appropriate light sources, such as a 405 nm laser and a 488 nm laser, were selected for intersection, voxel luminescence can be observed, and the luminescence intensity was high. The reaction scheme is as follows:

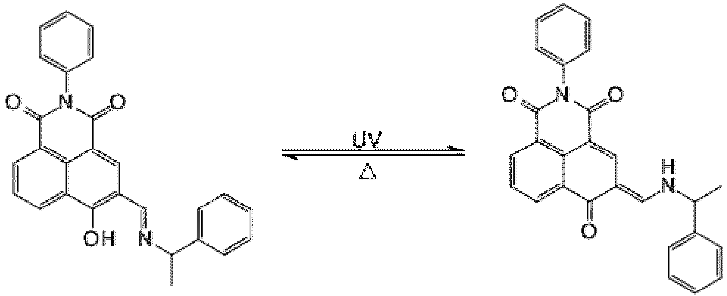

By controlling dual-beam scanning for addressing within the solution, volumetric three-dimensional display can be achieved. Accordingly, the naphthalimide-based Schiff base derivative obtained in Example 1 can be applied to a beam-addressable static volumetric three-dimensional display system. Therefore, it can be seen that the naphthalimide-based Schiff base derivative of the present disclosure can enhance the luminescence intensity of the keto isomer of the Schiff base, such that the derivative can serve as a display medium for static volumetric three-dimensional display, thereby improving the luminescence intensity of voxel points generated through light-beam addressing.

In the present disclosure, unless otherwise specified, terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present disclosure, "plurality" means at least two, such as two and three, unless otherwise specifically defined.

Reference throughout this specification to terms such as "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that above embodiments are merely exemplary, and cannot be construed to limit the present disclosure. For those skilled in the art, changes, alternatives, and modifications can be made to the embodiments without departing from the scope of the present disclosure.

## Claims

1. A naphthalimide-based Schiff base derivative, having a structural formula of: 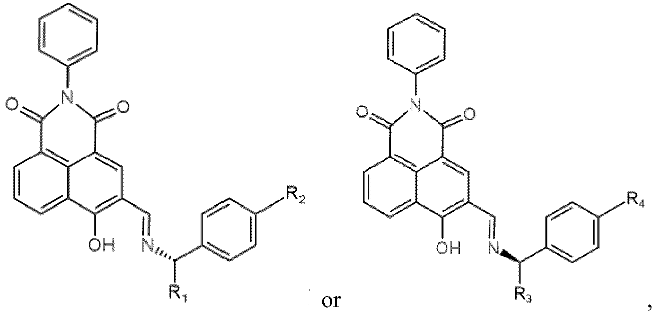 wherein R₁, R₂, R₃, and R₄ are each independently H or alkyl having 1 to 4 carbon atoms.

2. A method for preparing the naphthalimide-based Schiff base derivative according to claim 1, the method comprising:
performing a first reaction on a first compound with N-(3-formyl-4-hydroxy-1,8-naphthodicarboxyl)aniline in a first organic solvent to obtain the naphthalimide-based Schiff base derivative, wherein the first compound has a structural formula of: 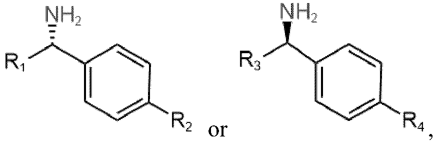
wherein R₁, R₂, R₃, and R₄ are each independently H or alkyl having 1 to 4 carbon atoms.

3. The method according to claim 2, wherein a molar ratio of N-(3-formyl-4-hydroxy-1,8-naphthodicarboxyl)aniline to the first compound is 1:(0.9 to 1.1).

4. The method according to claim 2 or claim 3, wherein the first organic solvent comprises at least one of methanol, ethanol, or dimethyl sulfoxide.

5. The method according to any one of claims 2 to 4, wherein N-(3-formyl-4-hydroxy-1,8-naphthodicarboxyl)aniline is prepared by:
step 1: performing a second reaction on 4-bromo-1,8-naphthalic anhydride with aniline in a first acidic solvent to obtain a second compound;
step 2: performing a third reaction on the second compound with a methoxy-containing reagent in a second organic solvent containing a catalyst to obtain a third compound;
step 3: performing a fourth reaction on the third compound with hydrohalic acid to obtain a fourth compound; and
step 4: performing a fifth reaction on the fourth compound with hexamethylenetetramine in a second acidic solvent to obtain N-(3-formyl-4-hydroxy-1,8-naphthodicarboxyl)aniline.

6. The method according to claim 5, wherein the first acidic solvent and the second acidic solvent each independently comprise glacial acetic acid and/or trifluoroacetic acid.

7. The method according to claim 5 or claim 6, wherein in step 1, a molar ratio of 4-bromo-1,8-naphthalic anhydride to aniline is 1:(1.5 to 3.5).

8. The method according to any one of claims 5 to 7, wherein in step 2, a molar ratio of the second compound, the methoxy-containing reagent, and the catalyst is 1:(7 to 8):(0.1 to 0.3).

9. The method according to any one of claims 5 to 8, wherein the second organic solvent comprises at least one of methanol, N,N-dimethylformamide, or dimethyl sulfoxide.

10. The method according to any one of claims 5 to 9, wherein the methoxy-containing reagent comprises sodium methoxide and/or potassium methoxide.

11. The method according to any one of claims 5 to 10, wherein the catalyst comprises at least one of copper sulfate, copper phosphate, copper acetate, or copper nitrate.

12. The method according to any one of claims 5 to 11, wherein in step 3, the hydrohalic acid comprises HI and/or HBr.

13. The method according to any one of claims 5 to 12, wherein a concentration of the hydrohalic acid is not less than 57 wt%.

14. The method according to any one of claims 5 to 13, wherein a content of the third compound in the hydrohalic acid ranges from 60 mmol/L to 100 mmol/L.

15. The method according to any one of claims 5 to 14, wherein in step 4, a molar ratio of the fourth compound to hexamethylenetetramine is 1:(1 to 3).

16. Use of the naphthalimide-based Schiff base derivative according to claim 1 in a static volumetric three-dimensional display system.
